## Europäisches Patentamt

## European Patent Office

## Office européen des brevets

(19)

(11) Veröffentlichungsnummer: **0 002 045**
**B1**

(12) **EUROPÄISCHE PATENTSCHRIFT**

(45) Veröffentlichungstag der Patentschrift:
02.09.81

(51) Int. Cl.³: **B 01 J 23/66, C 07 D 301/10**

(21) Anmeldenummer: 78101354.5

(22) Anmeldetag: 11.11.78

(54) **Verfahren zur Herstellung eines Äthylenoxydkatalysators.**

(30) Priorität: 19.11.77 DE 2751767
11.05.78 DE 2820520

(43) Veröffentlichungstag der Anmeldung:
30.05.79 Patentblatt 79/11

(45) Bekanntmachung des Hinweises auf die Patenterteilung:
02.09.81 Patentblatt 81/35

(84) Benannte Vertragsstaaten:
BE DE FR GB NL

(56) Entgegenhaltungen:
DE-A-2 521 906
FR-A-2 253 747
FR-A-2 359 642
US-A-3 962 136
US-A-4 010 115
US-A-4 012 425

(73) Patentinhaber: BASF Aktiengesellschaft,
Carl-Bosch-Strasse 38, D-6700 Ludwigshafen (DE)

(72) Erfinder: Mross, Wolf Dieter, Dr., Londoner Ring 75,
D-6700 Ludwigshafen (DE)
Erfinder: Titzenthaler, Eckart, Dr., Lenbachstrasse 11,
D-6700 Ludwigshafen (DE)
Erfinder: Koopmann, Juergen, Dr.,
Walter-Bruch-Strasse 48, D-6730 Neustadt 11 (DE)
Erfinder: Schwarzmann, Matthias, Dr.,
Carl-Bosch-Strasse 54, D-6703 Limburgerhof (DE)
Erfinder: Vogt, Volker, Dr., Carl-Bosch-Strasse 54,
D-6703 Limburgerhof (DE)

## Verfahren zur Herstellung eines Äthylenoxidkatalysators

Die Erfindung betrifft einen verbesserten Katalysator zur Herstellung von Äthylenoxid, der auf einem porösen Träger feinverteiltes Silber und als weitere Bestandteile Natrium und/oder Lithium sowie Kalium und/oder Rubidium und/oder Cäsium (»schwere Alkalimetalle«) enthält und bei dem auf den Träger zunächst Silber und gegebenenfalls Natrium bzw. Lithium in Form der betreffenden Salze aufgebracht und in üblicher Weise erhitzt (»aktiviert«) und in einer nachfolgenden Behandlung noch ein Salz eines schweren Alkalimetalls zusammen mit z. B. einem Amin und/oder Ammoniak aufgebracht wird. Die Bezeichnung »schweres Alkalimetall« bezieht sich dabei auf die Elemente Kalium, Rubidium und Cäsium.

Es ist bekannt, daß durch das Nachbehandeln eines gebrauchten alten Silberkatalysators (DE-AS 25 19 599) bzw. eines durch eine thermische Behandlung stabilisierten Silberkatalysators (US-PS 40 33 903) mit insbesondere Rubidium oder Cäsium die Selektivität eines Katalysators zur Bildung von Äthylenoxid aus Äthylen und Sauerstoff verbessert werden kann. Die Nachbehandlung soll geschehen, indem man einen Silberkatalysator, der eine Zeitlang zur Synthese von Äthylenoxid eingesetzt wurde und der sich dabei formiert bzw. stabilisiert hat, in dem Synthesereaktor mit einer Lösung eines der schweren Alkalimetalle in einem polaren organischen Lösungsmittel — vorgeschlagen wurde z. B. Methanol — imprägniert. Zur Verbesserung der Löslichkeit des Alkalisalzes kann das Lösungsmittel bis zu etwa 10% Wasser enthalten.

Behandelt man einen ungebrauchten bzw. nicht stabilisierten Silberkatalysator auf diese Weise, so erzielt man nahezu keine Selektivitätsverbesserung bzw. nur eine Selektivitätsverbesserung, die geringer ist als die, die man bei Einsatz eines vorher gebrauchten bzw. stabilisierten Katalysators erhält (s. Vergleichsbeispiel 1). Die Verbesserung der Katalysatorselektivität ist eine Aufgabe, die die Erfindung löst:

Es wurde gefunden, daß man einen gegenüber den bisher bekannten Nachbehandlungsverfahren bezüglich der Selektivität verbesserten Katalysator erhält, wenn man die Nachbehandlung mit einer methanolischen Lösung vornimmt, die wenigstens ein Salz der schweren Alkalimetalle Kalium, besonders aber Rubidium oder Cäsium, gegebenenfalls eine geringe Menge Wasser sowie erfindungsgemäß eine organische Verbindung enthält, die mit Silber(I)ionen Komplexsalze über ein Sauerstoff- und/oder Stickstoffatom bildet.

Zwar ist schon aus der US-PS 4 010 115 und 4 012 425 bekannt, Silber in Form einer Lösung, die komplexbildende Verbindungen enthält, auf den Träger aufzubringen, jedoch können auf diese Weise keine Katalysatoren, deren Wirksamkeit z. B. nachgelassen hat, wieder mit schweren Alkalimetallen angereichert werden, und das gleichzeitige Aufbringen von Silber und schweren Alkalimetallen führt auch bei neuen Katalysatoren nicht zur höchstmöglichen erreichbaren Aktivität und Selektivität dieser Katalysatoren.

Mit der erfindungsgemäßen Lösung kann man auch dann einen Katalysator mit ausgezeichneter Selektivität herstellen, wenn man einen ungebrauchten Katalysator, d. h. ein Katalysatorvorprodukt, damit behandelt. Der Nachbehandlungsschritt muß demnach auch nicht im Synthesereaktor erfolgen, sondern es kann die Herstellung des Silberkatalysators einschließlich Nachbehandlung in einer zur Herstellung von Trägerkatalysatoren üblichen geeigneten Apparatur durchgeführt werden. Solche Apparate und deren Verwendung sind z. B. in Ullmann's Enzyklopädie der technischen Chemie, 3. Aufl., Bd. 13, S. 517 ff., beschrieben.

Unter dem Begriff »gebrauchter« bzw. »ungebrauchter, aber nicht nachbehandelter« Katalysator soll im folgenden ein Katalysator verstanden werden, der auf einem handelsüblichen Träger (z. B. Aluminiumoxid, Kieselsäure oder einem Silikat) 2 bis 12, insbesondere 5 bis 10 Gewichtsprozent elementares Silber und, bezogen auf den Silberanteil, gegebenenfalls bis zu 2 Atomprozent Natrium oder Lithium bzw. deren Mischungen enthält sowie gegebenenfalls von einer früheren Behandlung her noch geringe (weniger als etwa 0,1 Atomprozent) Mengen an »schweren« Alkalimetallen.

Die erfindungsgemäß erzielbare Verbesserung wird erreicht, unabhängig davon, ob der zu behandelnde Katalysator Natrium oder Lithium enthält oder nicht, jedoch ist es besonders günstig, wenn der nach der Nachbehandlung vorliegende Katalysator etwa (bezogen jeweils auf Silber) 0,1 bis 2 Atomprozent Natrium und/oder Lithium sowie entweder 0,05 bis 0,35 Atomprozent Kalium, 0,003 bis 0,25 Atomprozent Rubidium oder 0,0005 bis 0,2 Atomprozent Cäsium oder eine Mischung der zuletzt genannten Alkalimetalle in entsprechender Menge enthält.

Zur Herstellung erfindungsgemäß verwendbarer Lösungen wird i. a. die Konzentration an »schweren« Alkalimetallen derart gewählt, daß sich nach dem Aufsaugen eines Teils der Lösung in den Katalysator und Entfernen des nicht aufgesaugten Teils die erfindungsgemäß wirksame Menge an Alkalimetallen auf dem Katalysator befindet.

Komplexbildner für Silber sind, wie aus Gmelins »Handbuch der anorganischen Chemie« (8. Aufl., Band Silber, Teil B 6, Springer-Verlag Berlin, Heidelberg, New York) hervorgeht, Verbindungen, die Sauerstoff oder Stickstoff enthalten. Ausgenommen sind natürlich solche Verbindungen, die bekannterweise Silberkatalysatoren schädigen, z. B. Phosphor- und Schwe-

felverbindungen.

Beispiele für geeignete, stark komplexbildende Sauerstoffverbindungen sind Verbindungen, die mehrere Sauerstoffatome in einer solchen Zuordnung enthalten, daß sie mit Silber Koordinationskomplexe bilden können. Geeignete Sauerstoff-Verbindungen sind z. B. Diketone wie Diacetyl oder Acetessigester bzw. Acetylaceton und mehrfache Äther, insbesondere die sog. Kronenäther.

Beispiele für Stickstoffverbindungen, die stark komplexbildend wirken, sind z. B. Amine, Ammoniak und Cyanverbindungen (Nitrile), wie z. B. Acetonitril. Als Amine im Sinne der Erfindung werden vorzugsweise aliphatische Amine, d. h. Mono-, Di- oder Trialkylamine, Alkanolamine und mehrwertige Amine wie die Alkylendiamine und Piperazine verwendet.

Die Komplexbildner werden z. B. in einer Menge von bis zu 30 Vol.-%, bezogen auf die für die Nachbehandlung vorgesehene Lösung, verwendet. Günstig ist eine Menge von 0,1 bis 10 Vol.-%.

Die Überführung des behandelten Katalysators bzw. Vorprodukts in die aktive Form geschieht wie üblich durch Erhitzen, gegebenenfalls in Gegenwart des üblichen gasförmigen Reaktionsgemisches aus Äthylen und Sauerstoff.

Die angegebene Zusammensetzung von Gasmischungen in den Beispielen bezieht sich wie üblich auf das Volumen.

### Beispiel 1

139 g Silbernitrat werden in einem Gemisch von 120 g sec.-Butylamin und 4 g in einer wäßrigen Lösung von 0,57 g Lithium- und 0,38 g Natriumnitrat gelöst. Die Lösung wird auf ein Volumen aufgefüllt, das der Flüssigkeitsaufnahmefähigkeit des vorgesehenen Trägers entspricht. Mit der vorbereiteten Lösung werden 1 kg Aluminiumoxid in Kugelform (Träger der Firma NORTON, Akron, U.S.A., Typ SA 5551) imprägniert. Das erhaltene Katalysatorvorprodukt wird nach einer Lagerung von einem Tag bei Raumtemperatur in einem Umluftofen bei 220°C getrocknet und dabei das Silber auf dem Träger abgeschieden. Der Katalysator enthält 8,0 Gewichtsprozent Silber, 0,55 Atomprozent Natrium und 1,0 Atomprozent Lithium. Der Katalysator erhält die Bezeichnung A.

10 ml zerkleinerter Katalysator A werden in einen Testreaktor mit 5 mm Innendurchmesser eingebaut und bei 15 bar Druck 30 Nl/h eines Gasgemisches, bestehend aus 28% Äthylen, 8% Sauerstoff, $2 \cdot 10^{-4}$ Vol.-% Vinylchlorid, Rest Stickstoff, durch den Katalysator geschickt. Die Temperatur wird so eingeregelt, daß ein 50%iger Sauerstoffumsatz erhalten wird. Nachdem nach einigen Tagen ein konstantes Aktivitätsniveau erreicht ist, wird der Versuch unterbrochen und der Katalysator im Reaktor bei Raumtemperatur mit einer methanolischen Lösung von $400 \cdot 10^{-4}$

Vol.-% $CsNO_3$, 5 Vol.-% sec.-Butylamin und 0,5 Vol.-% Wasser getränkt. Nach einstündiger Einwirkung wird die überschüssige Lösung abgelassen und durch Durchleiten von Stickstoff und Aufheizen des Reaktors der Katalysator getrocknet. Der so behandelte Katalysator erhält die Bezeichnung B. Mit dem vorher verwendeten Gasgemisch wird ein Aktivitäts- und Selektivitätstest bei 50%igem $O_2$-Umsatz durchgeführt. Das Ergebnis ist in der Tabelle 1 aufgenommen.

### Beispiel 2

100 ml Katalysator A werden mit einer methanolischen Lösung, die $350 \cdot 10^{-4}$ Vol.-% CsOH und 2 Vol.-% konzentrierten wäßrigen Ammoniak enthält, imprägniert und anschließend bei 220°C im Umluftofen getrocknet. Der erhaltene Katalysator (Bezeichnung C) wird dem Aktivitäts- und Selektivitätstest unterworfen (Tabelle 1).

### Beispiel 3

Man verfährt wie vorstehend beschrieben; die Imprägnierlösung enthält $330 \cdot 10^{-4}$ Vol.-% $CsCO_3$, 2 Vol.-% Äthylendiamin, 1 Vol.-% konzentrierten wäßrigen Ammoniak in Methanol; der Katalysator wird mit dem Buchstaben D gekennzeichnet. Das Ergebnis des Leistungsversuchs ist der Tabelle 1 zu entnehmen.

### Vergleichsbeispiel 1

Die Herstellung geschieht wie in Beispiel 1, in der Tränklösung fehlt aber sec.-Butylamin. Katalysator M, Tabelle 1.

### Vergleichsbeispiel 2

Die Herstellung geschieht wie in Beispiel 2, in der Imprägnierlösung fehlt aber der Ammoniak. Katalysator N, Tabelle 1.

Tabelle 1

| Katalysator | Vergleichstemperatur[*] °C | Selektivität[*] % |
|---|---|---|
| B | 221 | 81,5 |
| C | 217 | 80,5 |
| D | 218 | 80,5 |
| M (Vergleich) | 223 | 78,5 |
| N (Vergleich) | 218 | 77,5 |
| A (Vergleich) | 217 | 76,5 |

[*] Bei 50%igem Sauerstoffumsatz.

### Beispiel 4

100 ml Katalysator A, wie er gemäß Beispiel 1 erhalten wurde, werden mit einer methanolischen Lösung von $350 \cdot 10^{-4}$ Vol.-% CsOH und 2,5 Vol.-% Acetonitril imprägniert und anschließend bei 220°C im Umluftofen getrocknet. Der Katalysator mit der Bezeichnung E wird dem Aktivitäts- und Selektivitätstest unterworfen (Tabelle 2).

### Beispiel 5

Die Herstellung des Silberkatalysators geschieht wie in Beispiel 1. Die Imprägnierlösung enthält aber nur Lithiumnitrat. Die Weiterbehandlung geschieht ebenfalls wie vorstehend angegeben; die Nachbehandlungslösung enthält zusätzlich zu $350 \cdot 10^{-4}$ Vol.-% $CsNO_3$ noch $350 \cdot 10^{-4}$ Vol.-% $NaNO_3$. Der Katalysator trägt die Bezeichnung F (Tabelle 2).

### Beispiel 6

Die Herstellung geschieht wie in Beispiel 4. Es wird aber statt Acetonitril 1 Vol.-% Acetylaceton eingesetzt. Der Katalysator erhält die Bezeichnung G (Tabelle 2).

### Beispiel 7

Die Herstellung geschieht wie in Beispiel 4. Es wird aber statt Acetonitril 3 Vol.-% Dicyclohexyl-18-crown-6 (Kronenäther) verwendet. Katalysator H (Tabelle 2).

### Tabelle 2

| Katalysator | Vergleichs-temperatur*) °C | Selektivität*) % |
|---|---|---|
| E | 218 | 81,5 |
| F | 214 | 82,0 |
| G | 224 | 81 |
| H | 225 | 81 |

*) Vgl. Tabelle 1.

### Patentansprüche

1. Verfahren zur Aktivierung oder Reaktivierung eines Katalysators zur Herstellung von Äthylenoxid durch Umsetzung von Äthylenoxid und Sauerstoff in der Gasphase, der auf einem Träger etwa 2 bis 12 Gew.-% elementares Silber und Alkalimetalle bzw. deren Verbindungen enthält und so hergestellt wurde, daß man zunächst Silber und gegebenenfalls Natrium und/oder Lithium auf den Katalysatorträger in Form einer Salzlösung aufbrachte und reduzierte und wobei wenigstens eines der Alkalimetalle Kalium, Rubidium oder Cäsium in Form einer alkoholischen Lösung aufgebracht wird, die in einer solchen Menge verwendet wird, daß die vom Katalysator aufgenommene Lösung einer Menge von (bezogen auf Silber) 0,05 bis 0,35 Atomprozent Kalium, 0,003 bis 0,25 Atomprozent Rubidium oder 0,0005 bis 0,2 Atomprozent Cäsium oder entsprechenden Mengen eines Gemisches dieser Alkalimetalle entspricht und im Falle eines schon gebrauchten Katalysators schon vorhandene Mengen der Alkalimetalle berücksichtigt werden, dadurch gekennzeichnet, daß man eine methanolische Lösung wenigstens eines der Alkalimetalle Kalium, Rubidium oder Cäsium verwendet, die eine organische Verbindung enthält, die mit Silber(I)ionen Komplexsalze über ein Sauerstoff- und/oder Stickstoffatom bildet.

2. Verfahren nach Anspruch 1, dadurch gekennzeichnet, daß man als Katalysatorträger Aluminiumoxid verwendet.

3. Verfahren nach Anspruch 1, dadurch gekennzeichnet, daß man als Komplexsalze bildende Verbindung ein Amin und/oder Ammoniak verwendet.

4. Verfahren nach Anspruch 1, dadurch gekennzeichnet, daß man als Komplexsalze bildende Verbindung ein Nitril verwendet.

### Claims

1. A process for the activation or re-activation of a catalyst for the manufacture of ethylene oxide by reaction of ethylene with oxygen in the gas phase, which catalyst contains from about 2 to 12% of elementary silver and alkali metals or their compounds on a carrier, and has been prepared by first applying silver and optionally sodium and/or lithium in the form of a salt solution to the catalyst carrier and then effecting reduction, at least one of the alkali metals potassium, rubidium or cesium being applied in the form of an alcoholic solution which is used in such an amount that the solution absorbed by the catalyst corresponds to an amount (based on silver) of from 0.05 to 0.35 atom percent of potassium, of from 0.003 to 0.25 atom percent of rubidium or of from 0.0005 to 0.2 atom percent of cesium or to a corresponding amount of a mixture of these alkali metals, taking due account of any amounts of alkali metals which may already be present in the case of an already used catalyst, characterized in that a methanolic solution of at least one of the alkali metals potassium, rubidium or cesium is used which contains an organic compound which forms complex salts, via an oxygen atom and/or a nitrogen atom, with silver(I) ions.

2. A process as claimed in claim 1, character-

en ce que l'on utilise en tant que composé formant des sels complexes un nitrile.
the compound which forms complex salts.

4. A process as claimed in claim 1, characterized in that a nitrile is used as the compound which forms complex salts.

## Revendications

1. Procédé pour activer ou réactiver un catalyseur de préparation de l'oxyde d'éthylène par réaction de l'oxyde d'éthylène et de l'oxygène en phase gazeuse, qui contient sur un support environ 2 à 12% en poids d'argent élémentaire et des métaux alcalins ou leurs composés et qui a été préparé en appliquant d'abord sur le support de catalyseur l'argent et le cas échéant du sodium et/ou du lithium sous forme d'une solution de sel et en réduisant, au moins un des métaux alcalins potassium, rubidium ou caesium ayant été appliqué à l'état de solution alcoolique utilisée en quantité telle que la solution absorbée par le catalyseur corresponde à une quantité de (par rapport à ized in that an aluminium oxide is used as the catalyst carrier.

3. A process as claimed in claim 1, characterized in that an amine and/or ammonia is used as l'argent) 0,05 à 0,35 atome-gramme % de potassium, 0,003 à 0,25 atome-gramme % de rubidium ou 0,0005 à 0,2 atome-gramme % de caesium ou les quantités correspondantes d'un mélange de ces métaux alcalins tenu compte, dans le cas d'un catalyseur déjà utilisé, des quantités de métaux alcalins déjà présentes, caractérisé en ce que l'on utilise une solution méthanolique d'au moins un des métaux alcalins potassium, rubidium ou caesium, qui contient un composé organique formant avec les ions argent-I, par l'intermédiaire d'un atome d'oxygène et/ou d'azote, des sels complexes.

2. Procédé selon la revendication 1, caractérisé en ce que l'on utilise en tant que support de catalyseur une alumine.

3. Procédé selon la revendication 1, caractérisé en ce que l'on utilise en tant que composé formant des sels complexes une amine ou l'amoniac.

4. Procédé selon la revendication 1, caracterisé